# EUROPEAN PATENT APPLICATION

(11) **EP 2 039 311 A1**
(43) Date of publication of application: **25.03.2009**
(21) Application number: 08164572.3
(22) Date of filing: 18.09.2008
(51) Int. Cl.: A61B 17/80

(54) **Orthopaedic Bone Plate and Spacer**

(30) Priority: 20.09.2007 US 858446
(71) Applicant: DePuy Products, Inc., Warsaw, IN 46581 (US)
(72) Inventor: Graham, Matthew, Ft Wayne, IN 46814 (US); Schulze, Dale, Ft Wayne, IN 46814 (US); Jenks, Phillip, Leesburg, IN 46538 (US)
(74) Representative: Belcher, Simon James

(57) **Abstract**

An orthopaedic implant comprises a bone plate, and a remodelable spacer for positioning between the bone plate and a fractured bone. The spacer comprises a plurality of sheets of extracellular matrix material.

## Description

The present invention relates orthopaedic implants which include a bone plate and an associated spacer.

Internal fixation is the procedure by which a bone plate is implanted in a patient to facilitate the repair and healing of a fractured bone. In such a procedure, the bone plate is secured to bone fragments to secure the fragments in a desired orientation relative to one another. The bone plate is typically secured to the bone fragments by the use of bone screws threaded into the bone fragments. In some cases, the bone plate also compresses the bone fragments together in order to facilitate healing.

The present invention provides an orthopaedic implant which includes a bone plate and a spacer which can be remodelled, which can be positioned between the bone plate and a fractured bone. The remodelable spacer includes a plurality of sheets of extracellular matrix material.

Preferably, the remodelable spacer has a plurality of screw holes formed in it which are aligned with a plurality of screw holes defined in the bone plate.

The remodelable spacer may be embodied as a strip implanted between the bone plate and the bone. The remodelable spacer may also be embodied as a wrap that is wrapped around the bone plate or a sleeve into which the bone plate is inserted.

The remodelable spacer may be vacuum dried or freeze dried to the bone plate.

The sheets of extracellular matrix material may be embodied as sheets of small intestinal submucosa.

The sheets of extracellular matrix material may be embodied as sheets of vertebrate small intestine submucosa, vertebrate liver basement membrane, vertebrate bladder submucosa, vertebrate stomach submucosa, vertebrate alimentary tissue, vertebrate respiratory tissue, or vertebrate genital tissue.

The sheets of extracellular matrix material may be embodied as sheets of bovine tissue, ovine tissue, or porcine tissue.

The implant of the invention can be used in a method of performing a bone plating procedure includes positioning a remodelable spacer having a plurality of sheets of extracellular matrix material in a surgical site proximate to a bone. The method also includes positioning a bone plate in the surgical site such that at least a portion of the remodelable spacer is positioned between the bone plate and the bone. The method also includes fastening the bone plate to the bone.

The bone plate may be screwed to the bone with bone screws. As such, both the remodelable spacer and the bone plate may be embodied to include at least one screw hole. The bone plate may be positioned in the surgical site such that the screw hole(s) of the bone plate is/are aligned with the screw hole(s) of the remodelable spacer.

The remodelable spacer may be wrapped around the bone plate prior to positioning the remodelable spacer in the surgical site. The remodelable spacer may be embodied as a sleeve into which the remodelable spacer is inserted prior to positioning the remodelable spacer in the surgical site.

The remodelable spacer may be positioned in direct contact with the bone. Likewise, the bone plate may be positioned in direct contact with the remodelable spacer.

According to another aspect, an implantable orthopaedic device includes a bone plate having an upper surface and a lower surface with a remodelable coating disposed on the lower surface of the bone plate. The remodelable coating includes an extracellular matrix material.

The remodelable coating may be embodied as a gel, emulsification, or paste.

The extracellular matrix material of the coating may include vertebrate small intestine submucosa, vertebrate liver basement membrane, vertebrate bladder submucosa, vertebrate stomach submucosa, vertebrate alimentary tissue, vertebrate respiratory tissue, and vertebrate genital tissue.

The extracellular matrix material of the coating may include bovine tissue, ovine tissue, and porcine tissue.

According to another aspect, an orthopaedic implant includes a remodelable spacer for use in a bone plating procedure. The remodelable spacer includes an elongated body having a number of sheets of an extracellular matrix material with a number of screw holes defined therein.

The sheets of extracellular matrix material may be embodied as sheets of small intestinal submucosa.

The sheets of extracellular matrix material may be embodied as sheets of vertebrate small intestine submucosa, vertebrate liver basement membrane, vertebrate bladder submucosa, vertebrate stomach submucosa, vertebrate alimentary tissue, vertebrate respiratory tissue, or vertebrate genital tissue.

The sheets of extracellular matrix material may be embodied as sheets of bovine tissue, ovine tissue, or porcine tissue.

Embodiments of the invention are described below by way of example with reference to the accompanying drawings, in which:
FIG. 1 is a plan view of a bone plate;
FIG. 2 is a plan view of a remodelable spacer;
FIG. 3 is a side elevational view of the remodelable spacer of FIG. 2;
FIGS. 4 and 5 are cross sectional views of the bone plate and the remodelable spacer positioned in the surgical site during an orthopaedic bone plating procedure;
FIG. 6 is a side elevational view of another remodelable spacer;
FIG. 7 is a plan view of the remodelable spacer of FIG. 6, note the bone plate is shown in phantom positioned in the spacer;
FIG. 8 is a cross sectional view taken along the line 8-8 of FIG. 7, as viewed in the direction of the arrows;
FIG. 9 is a side elevational view of another embodiment of a remodelable spacer wrapped around the bone plate;
FIG. 10 shows the remodelable spacer and the bone plate of FIG. 9 in a plan view; and
FIG. 11 is a cross sectional view taken along the line 11-11 of FIG. 10, as viewed in the direction of the arrows.

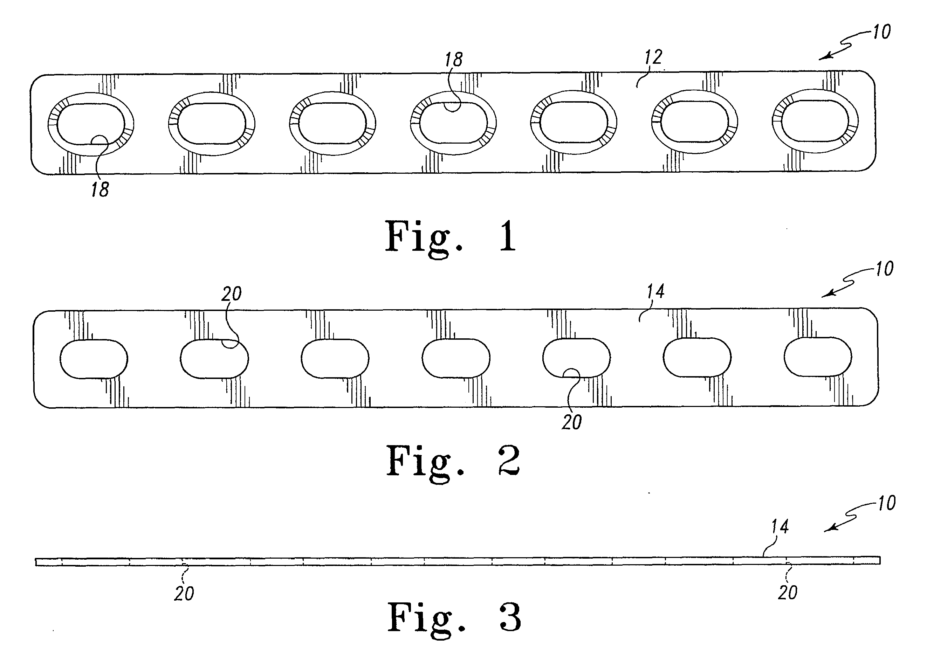

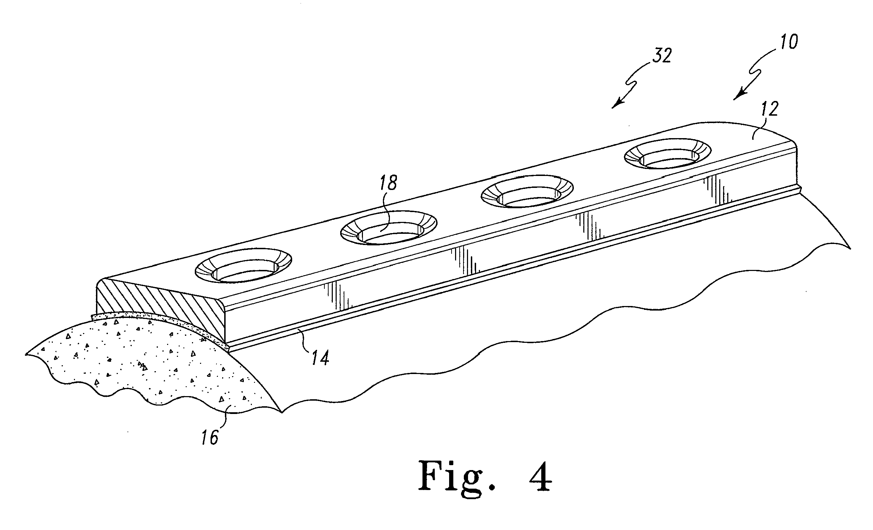

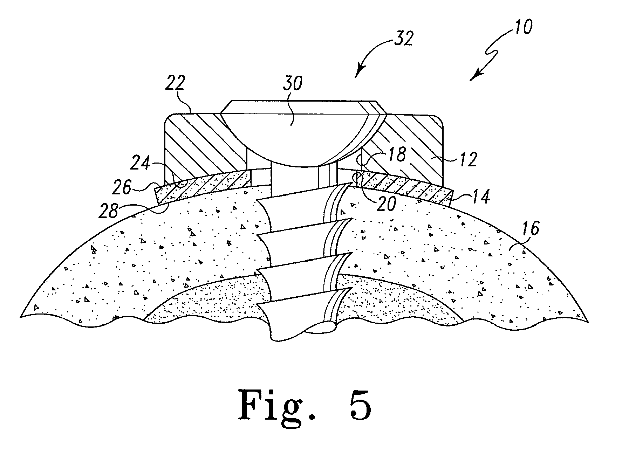

Referring to the drawings, FIGS. 1 to 5 show an orthopaedic implant 10 having a bone plate 12 and aremodelable spacer 14. As shown in FIGS. 4 and 5, the remodelable spacer 14 is positioned between the bone plate 12 and a bone 16 when the bone plate 12 is secured to the bone 16.

The bone plate 12 includes an elongated body having a number of screw holes 18 defined therein. The bone plate 12 is constructed of a rigid material such as metal or rigid polymers. In the illustrative embodiment described herein, the bone plate 12 is constructed of a medical implant-grade metal such as stainless steel or a titanium alloy.

The remodelable spacer 14 is illustratively embodied as a thin strip in the form of an elongated body having a number of screw holes 20 defined therein. In the illustrative embodiment described herein, the screw holes 20 of the remodelable spacer 14 align with the screw holes 18 of the bone plate 12 when the remodelable spacer 14 and the bone plate 12 are implanted into a surgical site 32 proximate to the bone 16.

In the embodiment shown in FIGS. 1 to 5, the remodelable spacer 14 includes as a plurality sheets of extracellular matrix material. As used in the fabrication of the remodelable spacer 14, extracellular matrices (ECMs) provide a scaffold for tissue repair and regeneration thereby accelerating the healing of the periosteum and promoting osteosynthesis of the fractured bone. In addition, use of an ECM in the fabrication of a remodelable spacer serves as a biological interface to protect the periosteum and reduces both stress-shielding and the risk of infection.

One such ECM is small intestine submucosa (SIS). Commercially available SIS material is derived from porcine small intestinal submucosa that remodels to the qualities of its host when implanted in human tissues. Further, it is taught that the SIS material provides a natural scaffold-like matrix with a three-dimensional microstructure and biochemical composition that facilitates host cell proliferation and supports tissue remodeling. Indeed, SIS has been shown to contain biological molecules, such as growth factors and glycosaminoglycans, that aid in the repair of tissue in the human body. SIS also contains antimicrobial polypeptides that inhibit bacterial growth in immediately surrounding tissues. SIS products, such as those sold under the trade marks OASIS and SURGISIS, are commercially available from Cook Biotech Inc., Bloomington, IN.

The orthobiologic implant SIS product sold byDePuy Orthopaedics Inc under the trade mark RESTORE is described for use during rotator cuff surgery, and is provided as a remodelable framework that allows the rotator cuff tendon to regenerate. The RESTORE Implant is derived from porcine small intestine submucosa, a naturally occurring ECM (composed of mostly collagen type I (about 90% of dry weight), glycosaminoglycans and other biological molecules) that has been cleaned, disinfected, and sterilized. During seven years of preclinical testing in animals, there were no incidences of infection transmission from the implant to the host, and the RESTORE Implant has not adversely affected the systemic activity of the immune system. The material used to make the RESTORE Implant, with or without modification thereto, may be used to fabricate the remodelable spacers described herein.

While small intestine submucosa is available, other sources of submucosa are known to be effective for tissue remodeling. These sources include, but are not limited to, stomach, bladder, alimentary, respiratory, and genital submucosa, and liver basement membrane, for example as referred to in US-6379710, US-6171344, US-6099567 and US-5554389. Further, while SIS is most often porcine derived, it is known that these various submucosa materials may be derived from non-porcine sources, including bovine and ovine sources. Additionally, the ECM material may also include partial layers of laminar muscularis mucosa, muscularis mucosa, lamina propria, stratum compactum layer and/or other such tissue materials depending upon other factors such as the source from which the ECM material was derived and the delamination procedure.

The terms "extracellular matrix" and "ECM", as used in this specification, include ECMs that have been cleaned and/or comminuted, or in which the collagen within the ECM has been crosslinked. However, it is not within the definition of "extracellular matrix" or "ECM" to separate and purify the natural fibres and reform a matrix material from purified natural fibres. Also, while reference is made to SIS, it is understood that other ECMs, such as stomach, bladder, alimentary, respiratory, or genital submucosa, or liver basement membrane, for example, whatever the source (e.g. bovine, porcine, ovine, etc.) are within the scope of this disclosure. Accordingly, the terms "extracellular matrix" or "ECM" are intended to refer to extracellular matrix material that has been cleaned, disinfected, sterilized, and optionally crosslinked.

Uses of ECMs for the regeneration and repair of various tissues are referred to in US-6379710, US-6187039, US-6176880, US-6126686, US-6099567, US-6096347, US-5997575, US-5993844, US-5968096, US-5955110, US-5922028, US-5885619, US-5788625, US-5733337, US-5762966, US-5755791, US-5753267, US-5711969, US-5645860, US-5641518, US-5554389, US-5516533, US-5460962, US-5445833, US-5372821, US-5352463, US-5281422, and US-5275826.

As alluded to above, the remodelable spacer 14 functions as a growth-supporting matrix for promoting tissue repair and regeneration thereby accelerating the healing of the periosteum and promoting osteosynthesis of the fractured bone. As such, when positioned adjacent a fractured bone, cells can migrate into and proliferate within the remodelable spacer 14, biodegrade the spacer 14 while, at the same time, synthesize new and healthy tissue to heal the fractured bone.

The remodelable spacer 14 may be configured any number of different ways to fit the needs of a given design. For example, as shown in the illustrative embodiment of FIGS. 1-5, the remodelable spacer 14 may be embodied as a plurality of sheets of ECM formed in the shape of an elongated strip having a thickness, for example, in the range of 0.5mm to 2mm. Such a plurality of sheets may be secured to one another in a number of different manners. In the exemplary embodiment described herein, the plurality of sheets of extracellular matrix material may be vacuum-dried or freeze-dried (for example by lypholisation) to one another. The illustrative remodelable spacer 14 is embodied as a plurality of sheets of SIS, each of which having been vacuum-dried to the other.

The remodelable spacer 14 may be prepared on site in the surgical room or prefabricated. Namely, the remodelable spacer 14 may be provided to the surgical room in the form of a strip of extracellular matrix material enclosed in a sterile package that is opened and cut into the desired shape with sterile scissors as part of the orthopaedic procedure. Alternatively, the remodelable spacer 14 may be die-cut by a manufacturer of the spacer 14. In such a case, the spacer 14 may be configured with a profile and hole pattern that mimics or compliments that of the bone plate 12. In such a case, the finished remodelable spacer 14 would likewise be provided to the surgery center in sterile packages. In the illustrative embodiment described herein, the remodelable spacer 14 is embodied as a prefabricated device which has been manufactured to closely compliment the bone plate 12.

The remodelable spacer 14 may be implanted as a separate component relative to the bone plate 12 during the orthopaedic procedure. Alternatively, the remodelable spacer 14 may be secured to the bone plate 12 prior to being implanted into the surgical site. Along this same line, the two components (i.e., the bone plate 12 and the remodelable spacer 14) may be secured to one another during fabrication. For example, the remodelable spacer 14 may be freeze-dried (for example by lypholisation) to the bottom surface 24 of the bone plate as part of the fabrication process. In such a case, the two components would be shipped to the surgical location in a single sterilized package.

For purposes of demonstrating an illustrative embodiment, reference is now made to FIGS. 4 and 5. In this case, the surgical site 32 proximate to the fractured bone 16 is first prepared in a conventional manner. Thereafter, the remodelable spacer 14 is introduced into the surgical site 32. To do so, the remodelable spacer is positioned on the bone 16 such that a lower surface 28 of the spacer contacts the outer surface of the bone 16, with the upper surface 26 of the remodelable spacer 14 facing upwardly. Although other configurations are contemplated, in the illustrative embodiment of the surgical method described herein, the bone plate 12 is positioned in the surgical site 32 such that it directly contacts the remodelable spacer 14. To do so, the bone plate 12 is implanted into the surgical site 32 and positioned in contact with the remodelable spacer 14 such that the lower surface 24 of the bone plate 12 contacts the upper surface 26 of the remodelable spacer 14. In such a configuration, the upper surface 22 of the bone plate faces away from the bone 16. Moreover, during such implantation of the bone plate 12, the plate 12 is positioned such that its screw holes 18 align with the screw holes 20 of the remodelable spacer 14.

Once implanted and positioned in such a manner, the bone plate 12 is fastened to the bone 16. Typically, this is done by the use of a plurality of bone screws 30. When the bone screws 30 are tightened, the remodelable spacer 14 is clamped between the bone plate 12 and the bone 16.

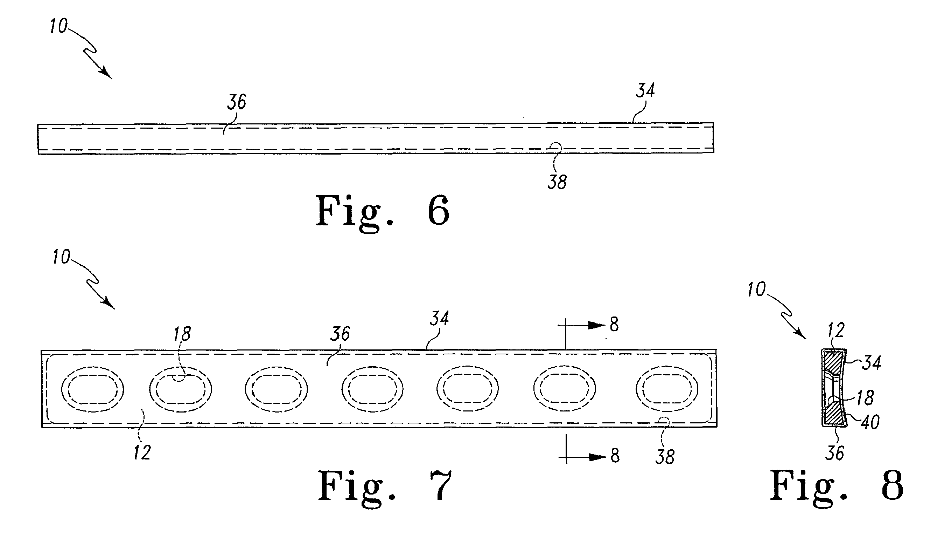

FIGS. 6 to 8 show a remodelable spacer 34 which can be fabricated from the same materials as the remodelable spacer 14 discussed above. The remodelable spacer 34 is embodied as a hollow sleeve. As such, the remodelable spacer 34 has a number of walls which define an interior void into which the bone plate 12 is inserted. Specifically, the remodelable spacer 34 has a number of side walls 36 which define an interior passageway 38. The bone plate 12 may be positioned in the passageway 38 prior to implantation of the bone plate 12 into the surgical site 32. In such a way, the bone plate 12 is enveloped by the remodelable spacer 36. In a similar manner to as a described above in regard to FIGS. 1 to 5, a lower wall 40 of the remodelable spacer 36 is clamped between the bone plate 12 and the bone 16 upon the insertion and tightening of the bone screws 30 during a plating procedure.

In the embodiment shown in FIG. 7, the upper wall of the remodelable spacer 34 does not have screw holes formed therein. However, in a similar manner to as described above in regard to the remodelable spacer 14 of FIG. 2, the upper wall 36 of the remodelable spacer 34 may have screw holes formed in it. Such screw holes may be formed on site in the surgery room, or they could be formed during fabrication of the remodelable spacer 34.

Similarly, the bone plate 12 may be inserted into the remodelable spacer 34 during the surgical procedure. In such a case, the bone plate 12 and the remodelable spacer 34 may be provided to the surgery room in separate sterilized packages. Alternatively, the bone plate 12 may be secured within the remodelable spacer 34 prior to arrival in the surgery room. For example, as part of the fabrication process, the remodelable spacer 34 may be vacuum-dried or freeze-dried (for example by lypholisation) to the bone plate 12 by the manufacturer.

As alluded to above, in a similar manner to the remodelable spacer 14 of FIG. 2, the remodelable spacer 34 may be constructed of a plurality of sheets of extracellular matrix material. Such a plurality of sheets of extracellular matrix material may be freeze-dried or vacuum-dried to one another. In the illustrative embodiment described herein, the remodelable spacer 34 is constructed of a plurality of sheets of SIS vacuum-dried to one another and formed into the shape of a sleeve.

Implantation of the orthopaedic implant of FIGS. 6 to 8 is similar manner to as described above in regard to FIGS. 1 to 5. Firstly, the bone plate 12 is inserted into the passageway 38 of the remodelable spacer 34. As described above, this could be done either in the surgery room or during manufacture of the orthopaedic implant. In either case, the remodelable spacer 34, with the bone plate 12 positioned therein, is then implanted into the prepared surgical site 32. Thereafter, the bone plate 12 is fastened to the bone 16 by use of, for example, a plurality of bone screws 30.

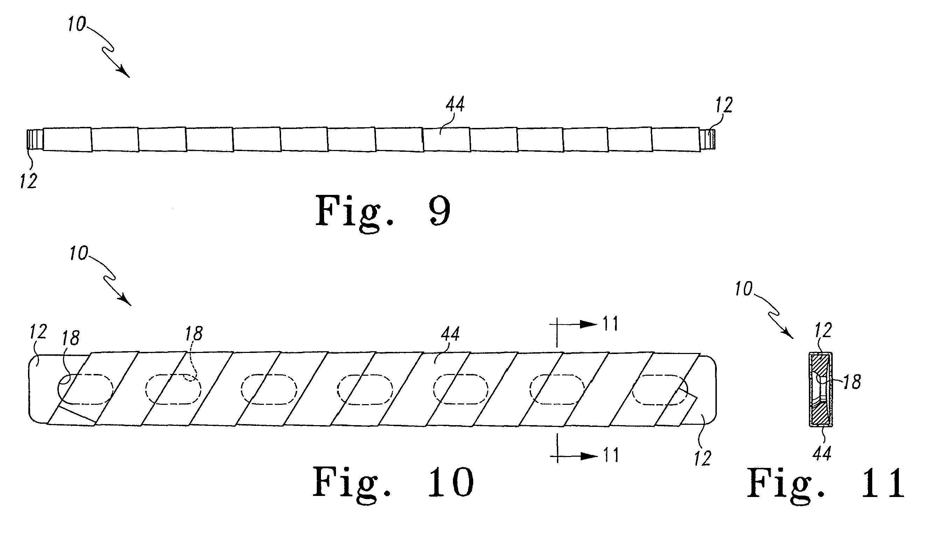

Another embodiment of a remodelable spacer 44 is shown in FIGS. 9 to 11. In this case, the remodelable spacer 44 is embodied as an elongated strip that is wrapped around the bone plate 12. Although the ends of the bone plate 12 are exposed in the illustrative embodiments of FIGS. 9 and 10, it should be appreciated that the remodelable spacer 44 may be sized to cover the entire bone plate.

As with other embodiments discussed above, the remodelable spacer 44 shown in FIGS. 9 to 11 may be constructed of a plurality of sheets of extracellular matrix material secured to one another. In this case, the elongated strip is wrapped around the bone plate 12 prior to implantation of the bone plate 12 into the surgical site 32. As such, like the other embodiments described herein, a lower surface 46 of the wrap is clamped between the bone plate 12 and the bone 16 when the bone plate 12 is fastened to the bone 16. As with the other embodiments described herein, the bone plate 12 may be secured to the bone 16 by use of a number of bone screws 30 advanced through the screw holes 18 of the bone plate 12. As such, the remodelable spacer 44 may be embodied with a number of screw holes. To do so, the screw holes could simply be cut into the remodelable spacer 44 with sterile scissors once the remodelable spacer 44 has been wrapped around the bone plate 12. Alternatively, screw holes may not be pre-cut into the spacer 44 with openings in the remodelable spacer 44 being formed when the bone screw 30 punctures the remodelable spacer 44 during plate attachment.

The remodelable spacer 44 may be snugly or loosely wrapped around the bone plate 12. In some embodiments, the remodelable spacer 44 may be vacuum-dried or freeze-dried to the bone plate 12 after it has been wrapped around the plate 12. Such could be done either in the surgery room or during implant manufacture.

As alluded to above, in a similar manner to the remodelable spacers 14, 34, the remodelable spacer 44 may be constructed of a plurality of sheets of extracellular matrix material. Such a plurality of sheets of extracellular matrix material may be freeze-dried or vacuum-dried to one another. In the illustrative embodiment described herein, the remodelable spacer 44 is constructed of a plurality of sheets of SIS vacuum-dried to one another and formed into the shape of an elongated strip.

Although the remodelable spacer 44 shown in FIGS. 9 to 11 can be used with many different types of bone plates, one particularly useful application for the remodelable spacer 44 is in conjunction with flexible bone plates such as those described in US-A-2008/0097432. That particular flexible bone plate includes a number of thin, stacked metallic laminates that collectively are relatively flexible until the plate is screwed tightly against the bone. In such an embodiment, the remodelable spacer 44 (or the remodelable spacer 34) may be used as a physical barrier that seals or otherwise reduces the accumulation of fluids between the laminates. In this case, as the ECM remodels into the periosteum, the compression of the flexible plate of the bone gradually decreases thereby allowing the bone plate to become gradually more flexible. This in turn reduces the stress shielding of the bone.

As described in relation to FIGS. 1 to 11, the remodelable spacer is embodied as a construct (e.g., strip, sleeve, or wrap) made from of a plurality of sheets of ECM secured to one another. Other embodiments of a remodelable spacer are also contemplated. For example, the remodelable spacer could be embodied as a remodelable coating disposed on the lower surface 24 of the bone plate 12. Such a coating could be fabricated by use of dry solids such as particulates, granules, beads, powder, or the like, mixed together with a sterile saline, blood, or other solvent. Such a composition may be coated onto the bottom surface 24 of the bone plate 12 prior to implantation of the bone plate 12 into the patient. Moreover, such a composition could be applied to the bottom surface 24 of the bone plate 12 during manufacture of the plate.

Further, a gel or emulsification of fabricated extracellular matrix material solids may be sprayed or brushed onto the bone plate 12 prior to implantation of the bone plate 12 into the patient. In a similar manner, a paste including ECM material solids may be formulated such that the surgeon could apply it in a similar manner to caulking prior to implanting the plate 12. Such a "caulk" could be used either in conjunction with, or in lieu of, one of the remodelable spacers described in relation to FIGS. 1 to 11. Such a gel, emulsification, paste may be fabricated with any suitable carrier compound which fits the needs of a given application, with techniques for making such compositions being known in the art.

## Claims

1. An orthopaedic implant, comprising:
a bone plate, and
a remodelable spacer configured to be positioned between the bone plate and a fractured bone, the remodelable spacer comprising a plurality of sheets of extracellular matrix material.

2. The orthopaedic implant of claim 1, in which the remodelable spacer has a plurality of screw holes formed in it which can be aligned with a plurality of screw holes defined in the bone plate.

3. The orthopaedic implant of claim 1, in which the remodelable spacer is wrapped around the bone plate.

4. The orthopaedic implant of claim 3, in which the remodelable spacer is vacuum dried to the bone plate.

5. The orthopaedic implant of claim 1, in which:
the remodelable spacer comprises a sleeve defining a interior void, and
the bone plate is positioned in the interior void of the sleeve.

6. The orthopaedic implant of claim 1, in which the plurality of sheets of extracellular matrix material comprises a plurality of sheets of small intestinal submucosa.

7. An implantable orthopaedic device, comprising:
a bone plate having an upper surface and a lower surface, and
a remodelable coating disposed on the lower surface of the bone plate, the remodelable coating comprising solids of extracellular matrix material.

8. The implantable orthopaedic device of claim 7, in which the remodelable coating comprises at least one of a gel, emulsification, or paste.

9. An orthopaedic implant, comprising:
a remodelable spacer for use in a bone plating procedure, the remodelable spacer having (i) an elongated body comprising a plurality of sheets of extracellular matrix material, and (ii) a number of screw holes defined in the body.

10. The orthopaedic implant of claim 9, in which the plurality of sheets of extracellular matrix material comprises a plurality of sheets of small intestinal submucosa.

11. The orthopaedic implant of any one of claims 1 to 10, in which the extracellular matrix material comprises tissue selected from the group consisting of:
vertebrate small intestine submucosa; vertebrate liver basement membrane; vertebrate bladder submucosa; vertebrate stomach submucosa; vertebrate alimentary tissue; vertebrate respiratory tissue; and vertebrate genital tissue.

12. The orthopaedic implant of any one of claims 1 to 11, in which the extracellular matrix material comprises tissue selected from the group consisting of: bovine tissue; ovine tissue; and porcine tissue.
